(19) Europäisches Patentamt
European Patent Office
Office européen des brevets

(11) Veröffentlichungsnummer: **0 437 555 B1**

(12) # EUROPÄISCHE PATENTSCHRIFT

(45) Veröffentlichungstag der Patentschrift: **30.08.95**

(51) Int. Cl.⁶: **C12P 41/00**, C07D 207/26

(21) Anmeldenummer: **90909039.1**

(22) Anmeldetag: **14.06.90**

(86) Internationale Anmeldenummer:
**PCT/DE90/00456**

(87) Internationale Veröffentlichungsnummer:
**WO 91/00362 (10.01.91 91/02)**

(54) **VERFAHREN ZUR RACEMATTRENNUNG VON 4-ARYL-2-OXO-PYRROLIDIN-3-CARBONSÄURE-ESTERN.**

(30) Priorität: **28.06.89 DE 3921593**

(43) Veröffentlichungstag der Anmeldung:
**24.07.91 Patentblatt 91/30**

(45) Bekanntmachung des Hinweises auf die Patenterteilung:
**30.08.95 Patentblatt 95/35**

(84) Benannte Vertragsstaaten:
**AT BE CH DE DK ES FR GB IT LI LU NL SE**

(56) Entgegenhaltungen:
**EP-A- 8 645**
**EP-A- 197 474**

**JOURNAL OF MEDICINAL CHEMISTRY, Band 32, Nr. 7, Juli 1989, American Chemical Society; M.C. MARIVET et al., Seiten 1450-1457/**

(73) Patentinhaber: **SCHERING AKTIENGESELLSCHAFT**
**Müllerstrasse 170/178**
**D-13353 Berlin (DE)**

(72) Erfinder: **PETZOLDT, Karl**
**Flachsweg 10**
**D-1000 Berlin 38 (DE)**
Erfinder: **SCHMIECHEN, Ralph**
**Bayernring 27**
**D-1000 Berlin 42 (DE)**
Erfinder: **HAMP, Kurt**
**Gloedenpfad 4**
**D-1000 Berlin 13 (DE)**
Erfinder: **GOTTWALD, Matthias**
**Zehntwerderweg 303A**
**D-1000 Berlin 28 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 437 555 B1

## Beschreibung

Die Erfindung betrifft ein Verfahren zur Racemattrennung von 4-Aryl-2-oxo-pyrrolidin-3-carbonsäureestern der allgemeinen Formel I

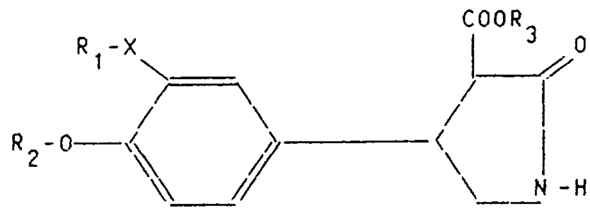

(I),

worin

X eine Kohlenstoff-Kohlenstoffbindung oder ein Sauerstoffatom darstellt,

$R_1$ einen gegebenenfalls durch Hydroxygruppen, Oxogruppen und/oder Halogenatome substituierten und/oder Stickstoffatome unterbrochenen Kohlenwasserstoffrest mit maximal 16 Kohlenstoffatomen bedeutet.

$R_2$ eine Alkylgruppe mit bis zu 4 Kohlenstoffatomen symbolisiert und

$R_3$ eine Alkylgruppe mit maximal 6 Kohlenstoffatomen darstellt, welches dadurch gekennzeichnet ist, daß man die racemischen 4-Aryl-2-oxo-pyrrolidin-3-carbonsäureester in wässriger Phase mit Pankreatin oder Cholesterin-Esterase umsetzt und die gebildete optisch aktive 4-Aryl-2-oxo-pyrrolidin-3-carbonsäure der allgemeinen Formel II

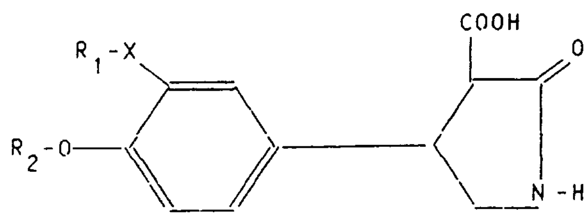

(II)

worin X, $R_1$ und $R_2$ die oben genannte Bedeutung besitzen und/oder den nicht umgewandelten optisch aktiven 4-Aryl-2-oxo-pyrrolidin-3-carbonsäureester der allgemeinen Formel I in an sich bekannter Weise isoliert.

In der EP A 0 197 474 wird ein Verfahren zur enzymatischen Razemattrennung von 2.3-Dihydroindol-2-carbonsäureestern beschrieben. Diese Verbindungen sind von ihrer Struktur her offensichtlich aber wesentlich geeigneter zur enzymatischen Razemattrennung als die Verbindungen der allgemeinen Formel II. Die so hergestellten optisch aktiven 4-Aryl-2-oxo-pyrrolidin-3-carbonsäuren der allgemeinen Formel II und/oder der optisch aktiven 4-Aryl-2-oxo-pyrrolidin-3-carbonsäureestern der allgemeinen Formel I können zur Herstellung von optisch aktiven 4-Aryl-2-pyrrolidonen der allgemeinen Formel III

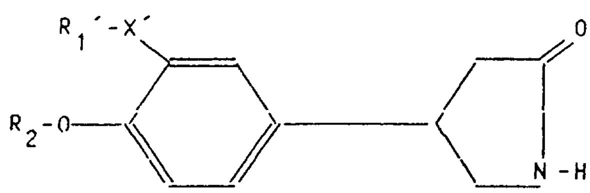

(III),

worin $R_1$'X' die gleiche Bedeutung wie $R_1X$ besitzt oder eine Hydroxygruppe darstellt und

$R_2$ eine Alkylgruppe mit bis zu 4 Kohlenstoffatomen symbolisiert verwendet werden.

4-Aryl-2-pyrrolidone der allgemeinen Formel III sind bekanntlich pharmakologisch wirksame Substanzen. So werden beispielsweise in der US-A 4,012,495 4-Aryl-2-pyrrolidone der allgemeinen Formel III mit $R'_1$ in der Bedeutung eines Kohlenwasserstoffs mit bis zu 18 Kohlenstoffatomen oder einer substituierten Alkylgruppe mit bis zu 5 Kohlenstoffatomen und X in der Bedeutung eines Sauerstoffatoms beschrieben,

die sich zur Behandlung neurologischer und psychischer Erkrankungen eignen. In der WO 86/02268 wird dargelegt, daß diese Substanzen zur topischen Behandlung von Entzundungen geeignet sind.

In der EP-B 0008645 werden 4-Aryl-2-pyrrolidone der allgemeinen Formel III mit $R'_1$ in der Bedeutung einer durch N-Heterocyclen substituierten Alkylgruppe oder Hydroxyalkylgruppe und X in der Bedeutung von Sauerstoff. Diese Substanzen zeichnen sich durch eine gefäßerweiternde und blutdrucksenkende Wirkung aus.

Erwähnenswert ist auch die DE-A 38 23 299, welche 4-Aryl-2-pyrrolidone der allgemeinen Formel III mit $R'_1$ in der Bedeutung eines aromatischen, nicht aromatischen oder heterocyclischen Ringsystems und X' in der Bedeutung einer Kohlenstoff-Kohlenstoffbindung betrifft. Diese Substanzen können als Wirkstoffe für Psychopharmaka Anwendung finden.

Von den 4-Aryl-2-pyrrolidonen der allgemeinen Formel III wurde das 4-[3-Cyclopentyl-4-methoxyphenyl]-2-pyrrolidon (=Rolipram) eingehend untersucht. Mittels eines sehr aufwendigen, im technischen Maßstab nicht nachvollziehbaren Verfahrens wurde diese Verbindung in ihre optischen Antipoden überführt und festgestellt, daß das (-)-4-[3-Cyclopentyl-4-methoxy-phenyl]-2-pyrrolidon die eigentliche pharmakologisch wirksame Komponente ist, während die entsprechende (+)-Antipode nur eine geringe Wirksamkeit zeigt.

Soweit bislang Ergebnisse vorliegen, ist auch bei anderen 4-Aryl-2-pyrrolidonendie (-)-Antipode pharmakologisch wesentlich stärker wirksam als die (+)-Antipode.

Da es wünschenswert ist, daß bei racemischen Arzneimittelwirkstoffen nur jeweils die wirksame Antipode zur Herstellung pharmazeutischer Präparate verwendet wird, bestand die Aufgabe, ein technisch problemlos durchführbares Verfahren zur Herstellung der optisch aktiven 4-Aryl-2-pyrrolidone der allgemeinen Formel III zu entwickeln. Diese Aufgabe wurde durch die Bereitstellung des erfindungsgemäßen Verfahrens gelöst.

Die als Ausgangsmaterialien für das erfindungsgemäße Verfahren dienenden 4-Aryl-2-oxo-pyrrolidin-3-carbonbsäureestern der allgemeinen Formel I können als Substituenten $R_1$ einen gegebenenfalls durch Hydroxygruppen, Oxogruppen und/oder Halogenatome substituierten und/oder durch Sauerstoffatome und/oder Stickstoffatome unterbrochenen Kohlenwasserstoffrest mit maximal 16 Kohlenstoffatomen tragen. Derartige Kohlenwasserstoffreste sind beispielsweise geradkettige oder verzweigte Alkylgruppen, Alkenylgruppen oder Alkinylgruppen mit maximal 8 Kohlenstoffatomen, Cycloalkylgruppen mit 3 bis 6 Kohlenstoffatomen, Cycloalkylalkylgruppen mit maximal 8 Kohlenstoffatomen, Phenylgruppen oder Benzylgruppen. Diese Kohlenwasserstoffreste können unsubstituiert sein oder die oben genannten Substituenten tragen. Als durch Sauerstoffatome unterbrochene Kohlenwasserstoffreste seien beispielsweise genannt Alkoxyalkylgruppen mit maximal 8 Kohlenstoffatomen, Alkoxyphenylgruppen und Alkoxybenzylgruppen mit maximal 4 Kohlenstoffatomen im Alkoxyrest oder die Tetrahydrofuranylgruppe. Als durch Stickstoffatome unterbrochene Kohlenwasserstoffreste seien beispielsweise genannt durch Piperidin, Morpholin oder Piperazin substituierte Alkylgruppen mit 2 bis 4 Kohlenstoffatomen, wobei der Heterocyclus seinerseits durch Phenylgruppen, Benzylgruppen oder auch Pyridylgruppen substituiert sein kann. Auch diese durch Sauerstoffatome und/oder Stickstoffatome unterbrochenen Kohlenwasserstoffreste können unsubstituiert sein oder durch Sauerstoffatome, Oxogruppen und/oder Halogenatome (vorzugsweise Fluor-oder Chloratome) substituiert sein.

Besonders bevorzugte Ausgangsmaterialien sind naturgemäß solche 4-Aryl-2-oxo-pyrrolidin-3-carbonsäureester der allgemeinen Formel I, die zu hoch aktiven Verbindungen der allgemeinen Formel III führen.

Derartige 4-Aryl-2-oxo-pyrrolidone der allgemeinen Formel III sind beispielsweise Verbindungen mit X in der Bedeutung eines Sauerstoffatoms, $R_1$ in der Bedeutung eines gesättigten oder ungesättigten, aliphatischen oder cyclischen Kohlenstoffrestes mit maximal 8 Kohlenstoffatomen (wie zum Beispiel der Ethylrest, der Isopropylrest, der 2-Propinylrest, der Cyclopropylmethylrest, der Cyclopentylrest oder der Benzylrest), $R_2$ in der Bedeutung einer Alkylgruppe mit bis zu 4 Kohlenstoffatomen insbesondere einer Methylgruppe, oder Verbindungen mit X in der Bedeutung eines Sauerstoffatoms, $R_1$ in der Bedeutung der Gruppierung

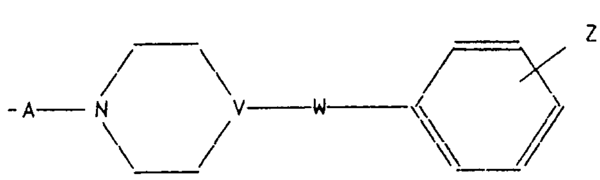

worin

A ein gegebenenfalls durch eine Hydroxygruppe substituierter Alkylenrest mit 2 bis 4 Kohlenstoffatomen,

V ein Stickstoffatom oder eine Methingruppe,

W eine N-C-Bindung, eine C-C-Bindung eine Methylengruppe oder eine Carbonylgruppe und

Z ein Wasserstoffatom oder ein Halogenatom symbolisieren, sowie $R_2$ in der Bedeutung einer Alkylgruppe mit bis zu 4 Kohlenstoffatomen, insbesondere einer Methylgruppe, oder Verbindungen mit X in der Bedeutung einer Kohlenstoff-Kohlenstoffbindung

$R_1$ in der Bedeutung eines gegebenenfalls durch 1 oder 2 Halogenatome substituierter Phenylrest oder Cycloalyklrest mit 3 bis 6 Kohlenstoffatomen, sowie $R_2$ in der Bedeutung einer Alkylgruppe mit bis zu 4 Kohlenstoffatomen, insbesondere einer Methylgruppe.

Bevorzugte Ausgangsmaterialien sind andererseits auch solche, die leicht spaltbare Gruppen R-X- (wie zum Beispiel die Benzyloxygruppe) enthalten. Aus diesen Substanzen lassen sich in einfacher Weise optisch aktive 4-Aryl-2-pyrrolidone der allgemeinen Formel III mit R'X'- in der Bedeutung einer OH-Gruppe herstellen, die ihrerseits mittels der bekannten Verfahren (US-A 4,012,495; EP-B 0008645 und DE-A 3823 299) in optisch aktive, pharmakologisch wirksame 4-Aryl-2-pyrrolidone überführt werden können.

Die als Ausgangsmaterial verwendeten 4-Aryl-2-oxo-pyrrolidin-3-carbonsäureester der allgemeinen Formel I können als REst $R_3$ eine Alkylgruppe mit maximal 6 Kohlenstoffatomen (vorzugsweise eine methylgruppe oder eine Ethylgruppe enthalten. Sie sind bekannt, oder können aus bekannten Vorprodukten mittels der Methoden hergestellt werden, die in der US-A 4,012,495 beschrieben sind.

Die racemischen 4-Aryl-2-oxo-pyrrolidin-3-carbonbsäureester der allgemeinen Formel I werden erfindungsgemäß in wässriger Phase mit Pankreatin oder Cholesterin-Esterase umgesetzt. Zu diesem Zweck wird das Pankreatin oder die Cholesterin-Esterase zweckmäßigerweise in wässiger Pufferlösung von pH 6 bis 10 (vorzugsweise bei pH 6 bis 8) gelöst, mit einer Lösung des Substrats in einem der üblicherweise verwendeten Lösungsmittel, wie Dimethylsulfoxid oder Dimethylformamid, versetzt und bei einer Reaktionstemperatur von 10 bis 40° C, vorzugsweise von 20-25° C gerührt bis 50 % des Substrates umgesetzt ist. Die Substratkonzentration beträgt üblicherweise 1 bis 20 g -vorzugsweise 1 bis 5 g pro Liter Fermentationsvolumen. Da das Pankreatin und die Cholesterin-Esterase je nach Herkunft eine unterschiedliche Zusammensetzung hat, muß die benötigte Menge Pankreatin oder Cholesterin-Esterase durch Vorversuche ermittelt werden, wie sie dem Fachmann geläufig sind.

Der Reaktionsablauf kann mittels der üblichen analytischen Techniken verfolgt werden.

Eine geeignete Methode ist beispielsweise die Analyse mittels Dünnschichtchromatographie oder Hochdruckflüssigkeitschromatographie (HPLC).

Bei den bislang durchgeführten Versuchen wurden fast ausschließlich Pankreatinpräparate und Cholesterin-Esterasen verwendet, die aus Schweinepankreas gewonnen waren, jedoch dürfte es sehr wahrscheinlich sein, daß auch Enzympräparate anderer Herkunft geeignet sind. Ein Versuch wurde mit einer Cholesterin-Esterase bakterieller Herkunft durchgeführt; dieses Enzym erwies sich als weniger geeignet.

Zur Durchführung des erfindungsgemäßen Verfahrens eignen sich beispielsweise folgende Enzympräparate:

Pankreatin-Granulat "Standard" der Firma Biochemie GmbH AT-Kundl; Produkt Nr. 461 173,
Pankreatin-Pulver der Firma Merck u. Co., DE-Darmstadt, ProduktNr. 7133.
Cholesterin-Esterase der Firma Fluka Chemie AG, CH-Buchs, Produkt Nr. 26745,
Cholesterin-Esterase der Firma Biozyme Lab. Ltd., GB-Bleanavon, Code CE-2 und
Cholesterin-Esterase der Firma Chemical Dynamics Corp., US-Plainfield (NJ), Produkt Nr. 22-2840-00.

Führt man das erfindungsgemäße Verfahren unter Verwendung von Cholesterin-Esterasen durch, so ist es zweckmäßig, der Reaktionsmischung ein nichtionisches Tensid wie zum Beispiel ein Alkylphenolethoxylat zuzusetzen, um das Enzym zu stabilisieren. Die Verwendung von Cholesterin-Esterasen hat den Vorzug, daß man diese in wesentlich geringeren Konzentration anwenden kann als Pankreatin. Darüberhinaus hat dieses Enzym den Vorteil, daß man es sehr gut zur Herstellung von Immobilisaten verwenden kann.

Die Immobilisierung des Enzyms durch Einbetten in ein dieses Enzym nicht denaturierendes Polymeres erfolgt nach Methoden, die dem Fachmann wohl bekannt sind. (I. Chibata Immobilized Enzymes; Reserch and Development 1978; S.P. Colowick und N.O. Kaplan, Methods in Enzymology, Academic Press, New York, et al. Vol. 44, 1976 und Bo Matthiasson Immobilised Cells and Organells, CRC Press Inc., Boca Raton, Florida Vol. 1 und 2.)

So kann man beispielsweise die Cholesterin-Esterase an aktivierter CH-Sepharose∫ der Firma Pharmacia Fine Chemicals, SE-Upsala oder an Acrylharzperlen Eupergit∫ oder Firma Röhm Pharma GmbH, DE-Darmstadt fixieren. Ein derartiges Immobilisat, in dem 100 mg Cholesterin-Esterase an 1 g Eurpergit∫ gebunden sind kann nach eigenen Untersuchungen mindestens in 20 Zyklen eingesetzt werden, ohne einen

signifikanten Aktivitätsverlust zu erleiden. Nach erfolgter Umsetzung kann das Immobilisat durch Filtration oder Zentrifugieren zurückgewonnen werden.

Nach erfolgter Umsetzung lassen sich die optisch aktive 4-Aryl-2-oxo-pyrrolidin-3-carbonbsäure der allgemeinen Formel II und der optisch aktive 4-Aryl-2-oxo-pyrrolidin-3-carbonbsäureester der allgemeinen Formel I mühelos voneinander trennen. Dies kann beispielsweise geschehen, indem man die Säure an basische Ionenaustauscher bindet, oder in dem man den Ester aus dem neutralen Reaktionsgemisch mittels der üblichen Lösungsmittel wie Essigsäureethylester, Methylisobutylketon oder Chloroform extrahiert, dann die Reaktionsmischung ansäuert und die freie Säure extrahiert. Ester und Säure können dann mittels der Methoden, die in der US-A 4,012,495 beschrieben sind in die optisch aktiven 4-Aryl-2-pyrrolidone der allgemeinen Formel III überführt werden.

Die nachfolgenden Ausführungsbeispiele dienen zur näheren Erläuterung des erfindungsgemäßen Verfahrens.

A. Beispiele betreffend das erfindungsgemäße Verfahren.

Beispiel 1

In einem 30 l fassenden Stahlfermenter werden 20 l Phosphatpuffer pH 7, hergestellt durch Auflösen von 145,24 g Dinatriumhydrogenphosphat-Dihydrat und 70,4 g Kaliumdihydrogenphosphat in 20 l Wasser, 40 Minuten bei 121° C sterilisiert. Anschließend wird die Pufferlösung auf 23° C abgekühlt. In einem 2 l-Erlenmeyerkolben weden 200 g "Pankreatin Granulat Standard", der Firma Biochemie Gesellschaft m.b.H Kundl/Tyrol/Austria, Materialnummer 00461173, eingewogen, mit 1,5 l aus dem Fermenter entnommener Pufferlösung mit einem Spatel kurz angerührt und sogleich in den gerührten Fermenter (100 Umdrehungen pro Minute) überführt). Anschließend wird noch 1 Minute durchgerührt und dann das Substrat, eine Lösung von 40 g 4-(3'-Cyclopentyloxy-4'-methoxy-phenyl)-2-oxo-pyrrolidin-3-carbonsäuremethylester in 400 ml Dimethylsulfoxid, hinzugegeben. Danach wird bei gleichbleibender Temperatur von 23° C die Rührgeschwindigkeit auf 220 Umdrehungen pro Minute erhöht. Nach 2 Stunden Rühren unter den genannten Bedingungen sind 50 % des eingesetzten Carbonsäureesters verseift. (Die Reaktion kann mittels Dünnschichtchromatographie an Kieselgel RP-Chiralplatten der Firma Macherey-Nagel u. Co. /DE 5610-Düren mittels Toluol-Essigester-Eisessig 70:15:15 verfolgt werden).

Jetzt wird der Ansatz aufgearbeitet, indem man das Reaktionsgemisch mit einem organischen Lösungsmittel in zwei Stufen extrahiert, wobei die entstandene (+)-Säure und der unverseifte (-)-Ester voneinander getrennt werden:

I. Das (neutrale) Reaktionsgemisch wird zunächst 2 mal mit je 20 l Methylisobutylketon extrahiert, die Extrakte vereinigt und im Vakuum zur Trockne eingeengt. Der verbliebene Rückstand kristallisiert langsam durch:

23,2 g(-)-4-(3'-Cyclopentyloxy-4'-methoxy-phenyl)-2-oxo-pyrrolidin-3-carbonsäuremethylester als krist. Rohprodukt (laut HPLC-Analyse 76,5 %ig, daraus errechnet sich die Ausbeute zu 44,4 % V.E.). Eine Probe wurde über eine Kieselgelsäule chromatographiert und danach aus Methylisobutylketon, anschließend aus Essigester umkristallisiert:

Schmlezpunkt 112-113° C, $(\alpha)_D^{20}$ -114,1° (c = 1 in Methanol)

II. Das extrahierte wässrige Reaktionsgemisch wird anschließend mit halbkonzentrierter Salzsäure auf pH 3 eingestellt und danach durch Filtration über eine Celiteschicht geklärt. Das Filtrat wird nun ebenfalls 2 mal mit je 20 l Methylisobutylketon extrahiert, die Extrakte vereinigt und im Vakuum zur Trockne eingeengt. Dabei hinterbleiben:

21,2 g(+)-4-(3'-Cyclopentyloxy-4'-methoxy-phenyl)-2-oxo-pyrrolidin-3-carbonsäure als bräunliches Ol (laut HPLC-Analyse 80,0 %ig, daraus errechnet sich die Ausbeute zu 44,5 % der Theorie).

Eine Probe wurde über eine Kieselgelsäule chromatographiert und anschließend aus Methanol kristallisiert:

Schmelzpunkt ca. 190° C (unspezifisch). $(\alpha)_D^{20}$ +93,4° (c = 1 in Methanol)

Beispiel 2

In einem 30 l-Stahlfermenter werden 20 l Phosphatpuffer pH 7 40 Minuten bei 121° C sterilisiert und danach auf 23° C temperiert.

In einem 2 l-Erlenmeyerkolben werden 200 g Pankreatin-Pulver der Firma Merck, Darmstadt, Artikelnummer 7133, eingewogen, mit 1,5 l aus dem Fermenter entnommener Pufferlösung mit einem Spatel angerührt und sogleich in den gerührten Fermenter (100 Umdrehungen pro Minute) überführt. Danach wird noch 1 Minute durchgerührt und dann das Substrat, eine Lösung von 20 g 4-(3'-Benzyloxy-4'-methoxy-phenyl-2-oxo-pyrrolidin-3-carbonsäureethylester in 200 ml Dimethylsulfoxid, hinzugegeben. Anschließend wird bei gleichbleibender Temperatur von 23° C die Rührgeschwindigkeit auf 220 Umdrehungen pro Minute erhöht. Nach 2 Stunden Rühren sind 50 % des eingesetzten Pyrrolidin-carbonsäureethylesters verseift.

Der Ansatz wird nun aufgearbeitet, indem man das Reaktionsgemisch in zwei Stufen extrahiert, wodurch die entstandene (+)-Pyrrolidin-carbonsäure vom unverseiften (-)-Pyrrolidin-carbonsäureethylester abgetrennt wird:

I. Das (neutrale) Reaktionsgemisch wird 2 mal mit je 2 ml Methylisobutylketon extrahiert, die Extrakte vereinigt und im Vakuum zur Trockne eingeengt. Es hinterbleiben 17,2 g eines ölig-kristallinen Rückstandes.

Zur Reinigung wird der Rückstand in 100 ml Essigester gelöst, mit 2 g Aktivkohle versetzt und 30 Minuten bei 50° C gerührt. Nach dem Abkühlen auf Raumtemperatur filtriert man die Mischung durch ein doppeltes Faltenfilter und engt das Filtrat im Vakuum zur Trockne ein. Der Rückstand wird in 100 ml Methylisobutylketon aufgenommen und über Nacht in der Tiefkühltruhe zur Kristallisation abgestellt:

$K_1$ = 9,4 g (-)-4-(3'-Benzyloxy-4'-methoxy-phenyl)2-oxo-pyrrolidin-3-carbonsäureethylester als weißes Kristallisat.

Eine Probe wurde noch 2 mal aus Essigester umkristallisiert: Schmelzpunkt 90-92° C, $(a)_D^{20}$ -87.0° (c=1 in Methanol)

II. Das extrahierte Reaktionsgemisch wird anschließend mit halbkonzentrierter Salzsäure auf pH 3 angesäuert und danach über Celite filtriert. Das Filtrat wird nun ebenfalls 2 mal mit je 20 l Methylisobutylketon extrahiert, die Extrakte vereinigt und im Vakuum zur Trockne eingeengt. Dabei hinterbleiben 13,4 g ölig kristalliner Rückstand.

Zur Reinigung wird der Rückstand in 100 ml 1n Natronlauge bei 50° C unter Rühren gelöst, auf Raumtemperatur abgekühlt und danach 3 mal mit je 50 ml Essigester extrahiert. Die organischen Phasen werden verworfen. Die wässrige Phase wird unter wird unter Rühren mit halbkonzentrierter Salzsäure langsam auf pH 3 gebracht, wobei die Pyrrolidin-carbonsäure ausfällt. Es wird 30 Minuten nachgerührt, der Niederschlag abgesaugt, anschließend in Essigester gelöst, mit Natriumsulfat getrocknet, filtriert und über Nacht zur Kristallisation in der Tiefkühltruhe abgestellt:

$K_1$ = 6,4 g (+)-4- (3'-Benzyloxy-4'-methoxy-phenyl)-2-oxo-pyrrolidin-3-carbonsäure als weißes Kristallisat.

Eine Probe wird ein weiteres Mal aus Essigester umkristallisiert:

Schmelzpunkt 145-146° C, $(\alpha)_D^{20}$ +105,2° (c = 1 in Methanol)

Beispiele betreffend das erfindungsgemäße Verfahren und die Verwendung der Verfahrensprodukte.

Beispiel 3:

In einem 10 l fassenden Glasfermenter werden 5 l Phosphatpuffer pH 7, hergestellt durch Auflösen von 36,31 g $Na_2HPO_4 2H_2O$ und 17,6 g $KH_2PO_4$ vollentsalztem Wasser, unter Zusatz von 15 Triton X-100 (ein Octylphenolethoxylat der Firma Fluka Chemie AG, CH-Buchs) (0,3 % v/v 45 Minuten bei 121° C sterilisiert. Nach Abkühlen der Lösung auf 23° C werden 100 ml Pufferlösung entnommen, in dieser 50 mg Cholesterin-Esterase aus Schweinepankreas der Firma Biozyme Laboratories Ltd., Bleanavon, South Wales, Great Britain, Code CE-2, suspendiert, die Suspension wieder in den Fermenter überführt und die Reaktionsmischung mit 220 Umdrehungen pro Minute gerührt. Direkt anschließend erfolgt die Zugabe des Substrates, einer Lösung von 10 g 4-(3'-Cyclopentyloxy-4'-methoxy-phenyl)-2-oxo-pyrrolidin-3-Carbonsäuremethylester (Rolipram-3-carbonsäuremethylester) in 100 ml Dimethylsulfoxid. Nach 72 Stunden Rühren bei 23° C sind 50 % des eingesetzten Substrates verseift.

Der Ansatz wird aufgearbeitet, indem man das Reaktionsgemisch mit einem organischen Lösungsmittel in zwei Stufen extrahiert, wobei die entstandere (+)-Rolipramsäure und der unverseifte (-)-Rolipramester voreinander getrennt werden:

Das neutrale Reaktionsgemisch wird zunächst 2 mal mit je 5 l Methylisobutylketion extrahiert, die Extrakte vereinigt und im Vakuum zur Trockne eingeengt. Zur Entfernung des mitextrahierten Triton X-100 wird der Rückstand mit etwa 400 ml Leitungswasser vermischt, 10 Minuten bei 50° C gerührt, über Nacht auf 20° C abgekühlt und anschließend abgesaugt. Es verbleibt ein kristallines Rohprodukt von 3,8 g (-)-4-(3'-Cyclopentyloxy-4'-methoxy-phenyl)-oxo-pyrrolidin-3-carbonsäure-methylester (nach HPLC 95 %ig, daraus berechnet sich eine Ausbeute zu 36 % V.E. ). Das Rohprodukt wird analog der in der US-A 4,012,495 für die Racemate beschriebenen Weise durch Verseifung und Decarboxylierung direkt in das (-)-4-(4'-Methoxy-phenyl)-2-pyrrolidon überführt. Das dabei entstehende Rohprodukt zeigt ohne weitere Aufreinigung bei Chromatographie in 96 %igem Ethanol an mikrokristallinem Cellulosetriacetat eine Reinheit von über 98

% Enantiomerenüberschuß.

Das extrahierte wässrige Reaktionsgemisch wird anschließend mit halbkonzentrierter Salzsäure auf pH 3 eingestellt und ebenfalls 2 mal mit je 5 l Methylisobutylketon extrahiert, die Extrakte vereinigt und im Vakuum zur Trockne eingeengt. Es verbleibt ein Rohprodukt von 7,12 g (+)-4-(3'-Cyclopentyloxy-4'-methoxy-phenyl)-2-oxo-pyrrolidin-3-carbonsäure als bräunliches Öl (nach HPLC 73,1 %ig, daraus errechnet sich eine Ausbeute zu 55 % d. Th.), das direkt durch Decarboxylierung in das (+)-4-(4'-Methoxy-phenyl)-2-Pyrrolidon überführt wird. Das dabei entstehende Rohprodukt hat nach Chromatographie an Cellulosetriacetat eine Reinheit von 92 % Enantiomerenüberschuß.

Beispiel 4:

100 mg Cholesterin-Esterase der Firma Biozyme Laboratories Ltd. werden in 4 ml eines 1 M Phosphatpuffers pH 7 suspendiert und zu 1 g Eupergit® C der Firma Röhm Pharma, D-6100 Darmstadt 1, gegeben. Nach kurzem Durchmischen läßt man den Ansatz für 48 Stunden bei 20° C stehen und entfernt anschließend nicht gebundenes Protein durch Waschen mit 0,1 M Phosphatpuffer über ein Glassinterfilter der Porosität 3, Das Immobilisat ist unter Zusatz von 0,02 % $NaN_3$ in Waschpuffer bei pH 7 und 4° C über mehrere Monate stabil.

Das Immobilisat wird unter Rühren in einen 10 l Glasfermenter mit 5 l sterilisiertem Phosphatpuffer, pH 7 gegeben. Anschließend erfolgt die Zugabe von 10 g 4-(3'-Cyclopentyloxy-4'-methoxy-phenyl)-2-oxo-pyrrolidin-3-carbonsäuremethylester (Rolipram-3-carbonsäuremethylester) in 100 ml Dimethylsulfoxid. Nach 136 Stunden sind etwa 50 % des eingesetzten Substrates umgesetzt.

Der Ansatz wird über ein Glassinterfilter abgesaugt und das Immobilisat bis zur weiteren Verwendung bei 4° C gelagert.

Das filtrierte Reaktionsgemisch wird aufgearbeitet, indem man es mit einem organischen Lösungsmittel in zwei Stufen extrahiert, wobei die entstandene (+)-Rolipramsäure und der unverseifte (-)-Rolipramester voneinander getrennt werden:

Das neutrale Reaktionsgemisch wird zunächst 2 mal mit je 5 l Methylisobutylketon extrahiert, die Extrakte vereinigt und im Vakuum zur Trockne eingeengt. Es verbleibt ein ölig-kristalliner Rückstand von 6,38 g (-)-4-(3'-Cyclopentyloxy-4'-methoxy-phenyl)-oxo-pyrrolidin-3-carbonsäure-methylester (nach HPLC 75,4 %ig, daraus errechnet sich eine Ausbeute von 48,1 %). Die optische Reinheit - bestimmt auf der Stufe des (-)-4-(4'-Methoxy-phenyl)-2-pyrrolidon - beträgt über 98 % Enantiomerenüberschuß.

Das extrahierte wässrige Reaktionsgemisch wird anschließend mit halbkonzentrierter Salzsäure auf pH 3 eingestellt und ebenfalls 2 mal mit je 5 l Methylisobutylketon extrahiert, die Extrakte vereinigt und im Vakuum eingeengt. Es verbleibt ein öliges Rohprodukt von 5,8 g (+)-4-(3'-Cyclopentyloxy-4'-methoxy-phenyl)-2-oxo-pyrrolidin-3-carbonsäure (nach HPLC 80 %ig, daraus errechnet sich eine Ausbeute von 46,4 %) . Die optische Reinheit - bestimmt auf der Stufe des (-)-4-(4'-Methoxy-phenyl)-2-pyrrolidon - beträgt 93 % Enantiomerenüberschuß.

Die für die in Beispielen beschriebenen enzymatischen Reaktionen benötigten Ausgangsverbindungen werden nach der in der DE 24 13 935 mit Beispielen belegten allgemeinen Reaktionsbeschreibung hergestellt. Ausbeuten (% d.Theorie) und Stoffdaten (Schmelzpunkt, Kristallisations-Lösungsmittel) sind in Tabelle 3 zusammengefaßt.

| Tabelle 3 | R' = -O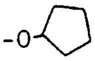 R= $-CH_3$ | R'= $-OCH_2$-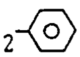 R = $-C_2H_5$ |
|---|---|---|
| 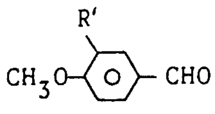 | langsam krist. Öl | Handelsprodukt<br>64° |
| 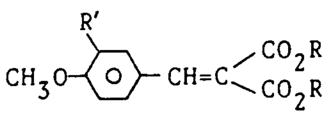 | 69 %<br>Öl | 86 %<br>84° (Ethanol) |
| 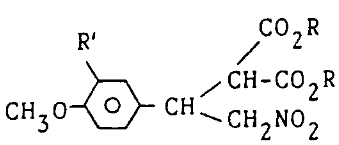 | 97 %<br>96-98° (Diisopropylether) | 91 %<br>80-82° (Ethanol/Diisopropylether) |
| 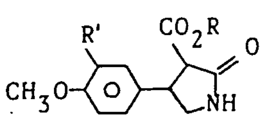 | 89 %<br>145-148° (Methanol) | 87 %<br>92-93° (Ethanol/Diisopropylether) |

Beispiele betreffend die Verwendung der Verfahrensprodukte

Die nach Beispiel 1 und 2 erhaltenen Pyrrolidon-3-carbonsäuren bzw. 3-Carbonsäureester werden analog der in der US-A 4,012,495 für die Racemate beschriebenen Weise durch Decarboxylierung bzw. Verseifung und Decarboxylierung in die Enantiomeren des 4-(4'-Methoxy-phenyl)-2-pyrrolidons (+)-I bzw. (-)-I überführt. (Daten siehe Tabelle 2)

Die Enantiomeren des 4-(3'-Benzyloxy-4'-methoxy-phenyl)-2-pyrrolidons (nach Beispiel 2) lassen sich in einfacher Weise durch hydrogenolytische Abspaltung der Benzylgruppe in die entsprechenden 3'-Hydroxyverbindungen überführen.

8,9 g (+)- oder (-)-4-(3'-Benzyloxy-4'-methoxy-phenyl)-2-pyrrolidon werden in 100 ml Ethanol gelöst und nach Zugabe von 0,5 g Palladium-Kohle (10 %ig) unter Schütteln bei Raumtemperatur und Normaldruck bis zum Stillstand der Wasserstoffaufnahme hydriert. Nach dem Abfiltrieren wird im Vakuum eingedampft und der Rückstand umkristallisiert (Daten des 4-(3'-Hydroxy-4'-methoxy-phenyl)-2-pyrrolidon siehe Tabelle 3, Nr. 1).

Aus den so hergestellten 3-Hydroxyverbindungen können in bekannter Weise durch Alkylierung die enantiomeren 4-(3'-Alkoxy-4'-methoxy-phenyl)-2-pyrrolidone US-A 4,012,495, EP-A 0008645) bzw. in Palladium katalysierter Reaktion der Trifluormethylsulfonate die enantiomeren 4-(3'-Cycloalkyl- bzw. 3'-Aryl-4'-methoxy-phenyl)-2-pyrrolidone (DE-P 38 23 299.5) synthetisiert werden. Einige repräsentative Beispiele sind in Tabelle 3 gegeben.

Tab. 2

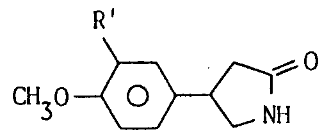

(+)-I / (-)-I

| R' | Ausbeute %d.Th. (+/-) | Kristallisations-Lösungsmittel | Schmelzpunkt (+/-) | $(\alpha)_D^{20}$ c=1, Methanol (+/-) |
|---|---|---|---|---|
| 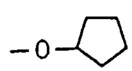 | 61,8 / 71.0 | Essigester | 133° / 133° | +33.8° / -33.0° |
| 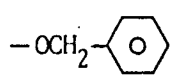 | 65,0 / 70.0 | Essigester / Diisopropylether | 137° / 137° | +30.1 / -29.7° |

Tab. 3

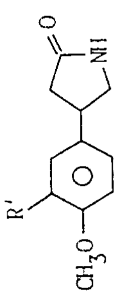

| R'- | Ausbeute %d.Th. (+/-) | Kristallisations-Lösungsmittel | Schmelzpunkt (+/-) | $(\alpha)_D^{20}$ (+/-) | c, Lösungsmittel |
|---|---|---|---|---|---|
| -OH | 92,0/90,7 | Methanol | 157-158°/157-158° | +35,9°/-35,8° | c=1; Pyridin |
| $-OCH_3$ | 67 / 77 | Essigester | 133° / 133° | +37,9°/-37,6° | c=1; Methanol |
| $-OCH_2C\equiv CH$ | 77 / 82 | Ethanol - Diisopropylether | 116° / 116° | +30,9°/-31,4° | c=1; Methanol |
| 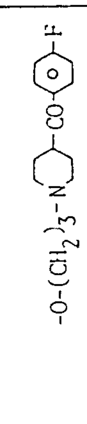 | 53,5/51,0 | Ethanol | 197-198°/197-198° | +19,5°/-18,5° | c=0,5; Methanol |
| 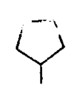 * | 40 / 43 | Essigester | 111° / 112° | +34,1°/-34,4° | c=0,5; Ethanol |

* übe. R' = $-OSO_2CF_3$

## Patentansprüche

1. Verfahren zur Racemattrennung von 4-Aryl-2-oxo-pyrrolidin-3-carbonsäure-estern der allgemeinen Formel I

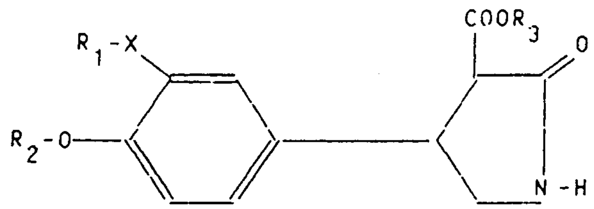

(I),

worin

X eine Kohlenstoff-Kohlenstoffbindung oder ein Sauerstoffatom darstellt,

$R_1$ einen gegebenenfalls durch Hydroxygruppen, Oxogruppen und/oder Halogenatome substituierten und/oder Stickstoffatome unterbrochenen Kohlenwasserstoffrest mit maximal 16 Kohlenstoffatomen bedeutet,

$R_2$ eine Alkylgruppe mit bis zu 4 Kohlenstoffatomen symbolisiert und

$R_3$ eine Alkylgruppe mit maximal 6 Kohlenstoffatomen darstellt,

dadurch gekennzeichnet, daß man die racemischen 4-Aryl-2-oxo-pyrrolidin-3-carbonsäureester in wässriger Phase mit Pankreatin oder Cholesterin-Esterase umsetzt und die gebildete optisch aktive 4-Aryl-2-oxo-pyrrolidin-3-carbonsäure der allgemeinen Formel II

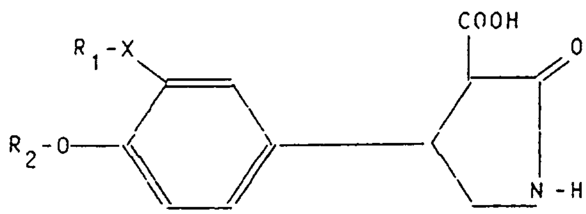

(II)

worin X, $R_1$ und $R_2$ die oben genannte Bedeutung besitzen und/oder den nicht umgewandelten optisch aktiven 4-Aryl-2-oxo-pyrrolidin-3-carbonsäureester der allgemeinen Formel I in an sich bekannter Weise isoliert.

## Claims

1. Process for the separation of the racemates of 4-aryl-2-oxo-pyrrolidine-3-carboxylic acid esters of the general formula I

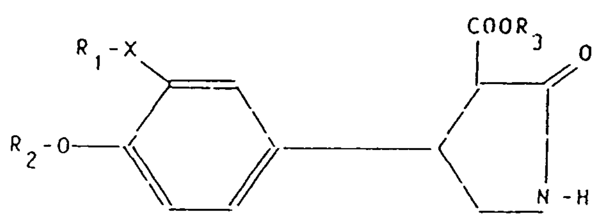

(I)

wherein

X represents a carbon-carbon bond or an oxygen atom,

$R_1$ represents a hydrocarbon radical having a maximum of 16 carbon atoms that is optionally substituted by hydroxy groups, oxo groups and/or by halogen atoms and/or that is optionally interrupted by nitrogen atoms,

$R_2$ represents an alkyl group having up to 4 carbon atoms, and

$R_3$ represents an alkyl group having a maximum of 6 carbon atoms,

characterised in that the racemic 4-aryl-2-oxo-pyrrolidine-3-carboxylic acid esters are reacted in the

aqueous phase with pancreatin or cholesterol esterase and the resulting optically active 4-aryl-2-oxo-pyrrolidine-3-carboxylic acid of the general formula II

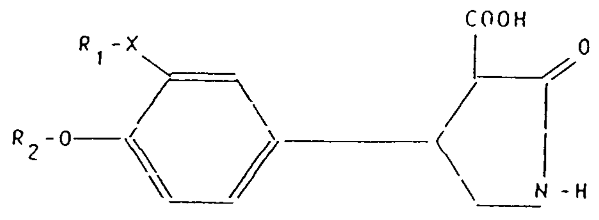

(II),

wherein X, $R_1$ and $R_2$ are as defined above, and/or the unconverted optically active 4-aryl-2-oxo-pyrrolidine-3-carboxylic acid ester of the general formula I, is/are isolated in a manner known per se.

**Revendications**

1. Procédé de séparation de racémates d'esters d'acides 4-aryl-2-oxo-pyrrolidine-3-carboxyliques de formule générale I

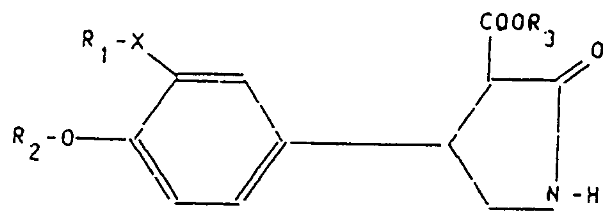

(I),

dans laquelle

X représente une liaison carbone-carbone ou un atome d'oxygène,

$R_1$ signifie un radical hydrocarboné ayant au maximum 16 atomes de carbone, éventuellement subsitué par des groupes hydroxyliques, oxo et/ou par des atomes d'halogènes et/ou interrompu par des atomes d'azote,

$R_2$ symbolise un alkyle ayant jusqu'à 4 atomes de carbone, et

$R_3$ représente un groupe alkyle avec au maximum 6 atomes de carbone,

procédé caractérisé en ce que l'on fait réagir les esters d'acides 4-aryl-2-oxo-pyrrolidine-3-carboxyliques racémiques en phase aqueuse avec de la pancréatine ou de la cholestérol-estérase et on isole de manière connue en soi l'acide 4-aryl-2-oxo-pyrrolidine-3-carboxylique optiquement actif ainsi formé, de formule générale II

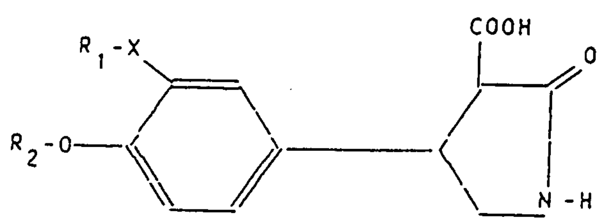

(II)

dans laquelle X, $R_1$ et $R_2$ possèdent la signification mentionnée ci-dessus et/ou l'ester de l'acide 4-aryl-2-oxo-pyrolidine-3-carboxylique optiquement actif non-transformé de formule générale I.